(19) Europäisches Patentamt

European Patent Office

Office européen des brevets

(11) Veröffentlichungsnummer: **0 187 386 B1**

(12) **EUROPÄISCHE PATENTSCHRIFT**

(45) Veröffentlichungstag der Patentschrift: 08.05.91

(21) Anmeldenummer: 85116637.1

(22) Anmeldetag: 28.12.85

(51) Int. Cl.⁵: **C07K 3/02**, C07K 3/20, //A61K37/02

(54) **Verfahren zur Isolierung und Reinigung von Lymphokinen.**

(30) Priorität: 11.01.85 DE 3500681

(43) Veröffentlichungstag der Anmeldung:
16.07.86 Patentblatt 86/29

(45) Bekanntmachung des Hinweises auf die
Patenterteilung:
08.05.91 Patentblatt 91/19

(84) Benannte Vertragsstaaten:
CH DE FR GB LI

(56) Entgegenhaltungen:
EP-A- 0 109 748
EP-A- 0 133 284
EP-A- 0 156 373
EP-A- 0 210 846
US-A- 4 405 601

(73) Patentinhaber: HOECHST AKTIENGESELL-
SCHAFT
Postfach 80 03 20
W-6230 Frankfurt am Main 80(DE)

(72) Erfinder: Müllner, Hubert, Dr.
Pestalozzistrasse 4
W-6233 Kelkheim (Taunus)(DE)

## EP 0 187 386 B1

## Beschreibung

Interleukin-2 oder der T-Zell-Wachstumsfaktor spielt eine wichtige zentrale Rolle im Ablauf einer Immunreaktion. Als Peptidsignal ermöglicht es eine Proliferation von Vorläuferzellen der cytotoxischen T-Zellen und auch die Proliferation von aktivierten B-Zellen (Lit.: Robb, Immunology Today 5 [1984] 203-209).

Bei allen Immundefizienzen bei denen ein Mangel an Interleukin-2 (IL-2) bzw. in der Biosynthese von IL-2 besteht, seien sie genetischer, erworbener oder iatrogener Art; kann IL-2 therapeutisch eingesetzt werden.

Das humane IL-2 vom Molekulargewicht 15 000, ist in der Sequenz charakterisiert (Lit.: Taniguchi et al., Nature 302 [1983] 305-309) und schon in E. coli cloniert worden.

Vom natürlichen, humanem IL-2 ist bekannt, daß es Zuckerreste besitzt, die aber für die Aktivität nicht essentiell sind. Aus einer Folge chromatographischer Schritte bestehende Reinigungsverfahren für Human-bzw. Primaten-IL-2 sind beispielsweise aus der DE-A1-32 42 851, aus der EP-A1-106 179 und aus Biochem. Biophys. Res. Commun. 115 [1983] 762-768 bekannt.

Beim rekombinanten IL-2 fehlen die Zuckerreste, die dem Molekül einen etwas hydrophileren Charakter geben. Daher neigt das IL-2 zur Aggregation. Besonders wenn es in E. coli in hoher lokaler Konzentration vorliegt, kommt es zur Aggregatbildung und damit zur Schwerlöslichkeit. Aus diesem Grund ist das in E. coli exprimierte IL-2 nach dem Aufschluß der Zellen (beispielsweise durch Entspannungsschock oder mit Hilfe von Enzymen wie Lysozym) im Niederschlag in einer schwerlöslichen Form zu finden. Durch Lösen und Ausfällen aus Guanidinium-HCl oder Harnstoff kann es zwar ebenfalls gereinigt werden, doch benötigt man dafür große Mengen stickstoffhaltiger Substanzen, deren Beseitigung Schwierigkeiten machen kann.

Es wurde nun überraschenderweise gefunden, daß sich der größte Teil des rekombinanten IL-2 im Niederschlag des Bakterienaufschlusses unter Verwendung einer gut lösenden, aber kaum denaturierenden, vorzugsweise eine organische Säure enthaltenden Lösungsmittelphase In Lösung bringen läßt. Die Isolierung des IL-2 kann in üblicher Weise, beispielsweise durch Gefriertrocknung erfolgen. Eine weitere Reinigung ist mittels chromatographischer Verfahren, vorzugsweise mittels Reverse-Phase-HPLC möglich.

Die Erfindung betrifft daher ein Verfahren zur Isolierung und Reinigung von Lymphokinen bzw. modifizierten Lymphokinen, vorzugsweise Interleukin-2 bzw. modifizierte Interleukine-2, das dadurch gekennzeichnet ist, daß man diese aus dem Sediment eines Zellaufschlusses mit einer organischen Lösungsmittelphase extrahiert und die Lymphokine bzw. modifizierten Lymphokine gegebenenfalls durch Chromatographie an einer unpolaren stationären Phase weiter reinigt.

Wie schon eingangs erwähnt, können die Lymphokine aus Kulturen von Human- und anderen Primatenzellen oder aus Kulturen von gentechnologisch veränderten Zellen isoliert werden. Bevorzugte Quelle für die Lymphokine bzw. modifizierten Lymphokine sind Kulturen gentechnologisch veränderter Zellen, vorzugsweise von Bakterien, insbesondere von E. coli.

Für das erfindungsgemäße Extraktionsverfahren haben sich Lösungsmittelphasen als geeignet erwiesen, die bis zu 100 % einer niederen aliphatischen Carbonsäure, vorzugsweise mit bis zu 4 C-Atomen enthalten. Besonders geeignete Extraktionsphasen enthalten eine niedere Alkansäure, vorzugsweise Essigsäure. Ein typisches Extraktionsmittel ist die reine aliphatische Carbonsäure oder ein Lösungsmittelgemisch, das diese Carbonsäure sowie Wasser, Acetonitril und/oder einen niederen aliphatischen Alkohol, vorzugsweise mit bis zu 4 C-Atomen enthält. Bevorzugte Alkohole sind gesättigte ein- oder mehrwertige Alkohole, insbesondere $(C_1-C_4)$-Alkanole, wie Ethanol. Durch Zugabe von Ammoniak oder flüchtigen organischen Aminen, wie Triethylamin, Morpholin oder Piperidin kann die Säure im Extraktionsgemisch abgepuffert werden.

Ist das Peptid in der beschriebenen Weise in Lösung gebracht, so kann es durch Chromatographie an einer unpolaren stationären Phase, vorzugsweise durch HPLC an hochporigem, alkyl-, aralkyl- oder arylsilyliertem Kieselgel weitergereinigt werden. Eine besonders bevorzugte stationäre Phase ist hochporiges Siliciumdioxid (Porenweite 25 - 35 nm (250 - 350 Å), vorzugsweise 30 - 33 nm (300 - 330 Å), das Alkylphasen von $C_4$ bis $C_{18}$ oder Phenyl gebunden hat.

Als Elutionsmittel verwendet man beispielsweise ein wäßriges System mit einem Gradienten a) eines niederen Alkylcyanids (vorzugsweise mit bis zu 4 C-Atomen, insbesondere Acetonitril), b) eines niederen aliphatischen, wie oben definierten Alkohols (vorzugsweise n-Propanol) oder c) des Gemisches davon in einem pH-Bereich von 1 bis 7, welcher z.B. mit Triflouressigsäure oder Tetraethylammoniumphosphat eingestellt wird.

Im Falle des rekombinanten IL-2 läßt sich auf diese Weise in einem Schritt eine 80 bis 95%ige Reinheit bei einer Ausbeute von ca. 80 bis 90 % erzielen.

Das erfindungsgemäße Verfahren ist zur Reinigung und Isolierung von Lymphokinen insbesondere solche, die die Aminosäuresequenz des Human-IL-2 aufweisen, und von modifiziertenLymphokinen, insbesondere solchen, die Teile der Aminosäuresequenz des Human-IL-2 aufweisen, geeignet.

2

Unter modifizierten Lymphokinen versteht man biologisch aktive Peptide, vorzugsweise mit der IL-2-Sequenz, deren Aminosäuresequenz modifiziert ist, indem

a) eine oder mehrere Aminosäuren durch andere, genetisch kodierbare Aminosäuren ausgetauscht sind, und/oder

b) N-terminal eine oder mehrere Aminosäuren fehlen oder

c) N-terminal eine oder mehrere genetisch kodierbare Aminosäuren zusätzlich vorhanden sind und/oder

d) C-terminal eine oder mehrere Aminosäuren fehlen oder

e) C-terminal eine oder mehrere genetisch kodierbaren Aminosäuren zusätzlich vorhanden sind.

Die vorstehend genannten Lymphokine bzw. modifizierten Lymphokine können weiterhin dadurch abgewandelt werden, daß eine oder mehrere Aminosäuren der Sequenz beispielsweise enzymatisch in genetisch nicht kodierbare Aminosäuren umgewandelt werden (z.B. Lys in Hyl) und/oder mit physiologisch verträglichen Gruppen, wie sie z.B. in Schröder, Lübke, The Peptides, Vol. I, Academic Press, New York 1965 beschrieben sind, substituiert sind.

Modifizierte IL-2 sind in der deutschen Patentanmeldung P 34 19 995.0 vorgeschlagen worden.

Die folgenden Beispiele dienen zur Erläuterung der Erfindung, ohne daß diese darauf beschränkt wäre.

Beispiel 1

Das nach dem Aufschluß der Pakterien erhaltene, gefriergetrocknete Sediment wurde in 50 %iger wäßriger Essigsäure aufgenommen, 10 Minuten gerührt, klar zentrifugiert und fein filtriert.

Beispiel 2

Das gemäß Beispiel 1 erhaltene Filtrat wurde direkt semipräparativ auf eine (R)Vydac-$C_{18}$-Widepore-Säule (Partikelgröße 10 $\mu$m, Durchmesser 1 cm, Länge 25 cm) aufgegeben. Die auf der Säule adsorbierten Peptide und Proteine wurden mit einem Trifluoressigsäure (TFA)/Acetonitril (AN)-Gradienten (in 20 min von 35 % Puffer B bis 85 % Puffer B) in einem Fluß von 2 ml/min eluiert (Puffer B = 0,1 % TFA, 80 % AN und 19,9 % $H_2O$). Das Interleukin-2 eluierte in einem scharfen Peak bei ca. 64 % Acetonitril.

Beispiel 3

Das gemäß Beispiel 1 erhaltene Filtrat wurde präparativ auf eine (R)Vydac-$C_{18}$-Widepore-Säule (Porenweite 33 nm (330 Å), Partikelgröße 15 - 20 $\mu$m, Durchmesser 2 cm, Länge 30 cm) aufgegeben und mit dem im Beispiel 2 beschriebenen Elutionsmittel in einem Fluß von 12 ml/min eluiert.

Beispiel 4

Das gemäß Beispiel 1 erhaltene Filtrat wurde analytisch auf eine (R)Biorad-Hipore-RP 318-Säule (Porenweite 30nm (300 Å), Partikelgröße 5 $\mu$m, Durchmesser 4 mm, Länge 250 mm) aufgegeben und in einem Fluß von 1,5 ml/min mit dem im Beispiel 2 beschriebenen Lösungsmittelsystem eluiert.

Das gemäß den Beispielen 2 bis 4 erhaltene IL-2 ist hochrein (besser als 90 %). Bei einer Rechromatographie von 25 $\mu$g IL-2 gemäß Beispiel 4, wurden 24 $\mu$g wieder eluiert. Das entspricht einer Ausbeute von ca. 95 %.

Zum Erhalt der Aktivität des rekombinanten Materials wird das gereinigte Material mit ca. 1 mg Human-Serumalbumin/ 100 $\mu$g IL-2 versetzt und gefriergetrocknet. Das Trägerprotein verhindert die Aggregation, führt zu einer leichten Löslichkeit und damit zum Erhalt der biologischen Aktivität.

Beispiel 5:

Extraktion von IL-2 mit Mischungen von Essigsäure und n-Butanol bzw. Propylenglykol

Mikroorganismen, die IL-2 enthielten wurden vermittels einer "French press" durch Entspannungsschock lysiert und das Homogenat zentrifugiert. Das Sediment wurde 1 x mit 0,1 m Phosphatpuffer pH 7,0 gewaschen und dann lyophilisiert.

3

Je 10 mg von diesem ersten lyophilisierten Sediment, das noch Membranfragmente, alle unlöslichen Proteine und in geringerem Maß noch Puffersalze enthält, wurden in 1 ml von verschiedenen Mischungen bestehend aus Essigsäure, n-Butanol oder Propylenglykol und Wasser suspendiert.

Nach 1 Minute des Schüttelns werden die Proben zentrifugiert und ein Aliquot des Überstands auf einer analytischen Biorad RP 318 (R)Hipore-HPLC-Säule chromatographiert wie schon beschrieben.

```
Extraktionsmischung / IL-2 Konz.              in mg/ml (HPLC gemessen)
─────────────────────────────────────────────────────────────────────
 1,50 %  Essigsäure                           0,01
 2,60 %       "                               0,054
 3,70 %       "                               0,110
 4,50 %       "      +10 % n-Butanol          0,076
 5,60 %       "        "        "             0,124
 6,70 %       "        "        "             0,138
 7,50 %       "       20 %      "             0,156
 8,60 %       "        "        "             0,156
 9,70 %       "        "        "             0,206
10,50 %       "      +10 % Propylenglykol     0,045
11,60 %       "        "        "             0,069
12,70 %       "        "        "             0,144
```

Reduktion des Extraktionsvolumens führt zu einem fast linearen Anstieg in der Konz. des extrahierten Interleukin 2 ohne dramatische Verschiebung des Verhältnisses von Interleukin 2/Verunreinigungen.

```
10 mg/ml Extr.    70 % Essigsäure    +20 % n-Butanol    0,2 mg/ml IL-2
40 mg/ml          70 %     "         +20 %     "        0,89      "
10 mg/ml          60 %     "         +30 %     "        0,2       "
20 mg/ml          60 %     "          30 %     "        0,44      "
50 mg/ml          60 %     "          30 %     "        1,06      "
```

Bei einer Vernachlässigung des Injektionsartefakts, der durch die Extraktionsmittel verursacht wird, aber auch stark hydrophile Verunreinigungen enthält, bewegt sich der Prozentsatz des IL-2-Peaks im Bereich von 30-40 %.

**Ansprüche**

1. Verfahren zur Isolierung und Reinigung von Lymphokinen bzw. modifizierten Lymphokinen aus Zellaufschlüssen von Kulturen gentechnologisch veränderter Bakterien, dadurch gekennzeichnet, daß man diese aus dem Sediment eines Zellaufschlusses mit einer niederen aliphatischen Carbonsäure oder einem diese Carbonsäure, sowie Wasser, Acetonitril und/oder einen niederen aliphatischen Alkohol enthaltenden Gemisch extrahiert und die Lymphokine bzw. modifizierten Lymphokine gegebenenfalls durch Chromatographie an einer unpolaren stationären Phase weiter reinigt.

2. Verfahren gemäß Anspruch 1, dadurch gekennzeichnet, daß man Lymphokine bzw. modifizierte Lymphokine aus E. coli isoliert und reinigt.

3. Verfahren gemäß Anspruch 1 oder 2, dadurch gekennzeichnet, daß man Essigsäure oder ein Essigsäure enthaltendes Gemisch verwendet.

4. Verfahren gemäß einem der Ansprüche 1 bis 3, dadurch gekennzeichnet, daß man die extrahierten Lymphokine bzw. modifizierten Lymphokine mittels HPLC an einem derivatisierten Kieselgel zur Reserve-Phase-Chromatographiereinigt.

5. Verfahren gemäß Anspruch 4, dadurch gekennzeichnet, daß man als stationäre Phase ein hochporiges, alkylsilyliertes, aralkylsilyliertes oder arylsilyliertes Kieselgel verwendet.

6. Verfahren gemäß Anspruch 4 oder 5, dadurch gekennzeichnet, daß man als Elutionsmittel ein wäßriges System mit einem Gradienten eines niederen Alkylcyanids, vorzugsweise Acetonitril, oder eines niederen Alkohols, vorzugsweise n-Propanol, oder des Gemisches davon in einem pH-Bereich von 1 bis 7 verwendet.

7. Verfahren gemäß einem der Ansprüche 1 bis 6, dadurch gekennzeichnet, daß man Interleukin-2 bzw. modifiziertes Interleukin-2 isoliert und reinigt.

8. Verfahren gemäß einem der Ansprüche 1 bis 7, dadurch gekennzeichnet, daß man ein Peptid isoliert und reinigt, das die Aminosäuresequenz des Human-Interleukins-2 aufweist.

9. Verfahren gemäß einem der Ansprüche 1 bis 7, dadurch gekennzeichnet, daß man ein biologisch aktives modifiziertes Lymphokin, vorzugsweise ein biologisch aktives modifiziertes Interleukin-2 isoliert und reinigt, dessen Aminosäuresequenz modifiziert, indem
   a) eine oder mehrere Aminosäuren durch andere, genetisch codierbare Aminosäuren ausgetauscht sind, und/oder
   b) N-terminal eine oder mehrere Aminosäuren fehlen oder
   c) N-terminal eine oder mehrere genetisch codierbare Aminosäuren zusätzlich vorhanden sind und/oder
   d) C-terminal eine oder mehrere Aminosäuren fehlen oder
   e) C-terminal eine oder mehrere genetisch codierbaren Aminosäuren zusätzlich vorhanden sind.

10. Verfahren gemäß Anspruch 8 oder 9, dadurch gekennzeichnet, daß eine oder mehrere Aminosäuren der Sequenz umgewandelt und/oder mit physiologisch verträglichen Gruppen substituiert sind.

**Claims**

1. A process for the isolation and purification of lymphokines or modified lymphokines from disruptions of cells from cultures of bacteria which have been modified by genetic engineering, which comprises extraction of them, using a lower aliphatic carboxylic acid or a mixture containing this carboxylic acid together with water, acetonitrile and/or a lower aliphatic alcohol, from the sediment from a cell disruption, and further purification of the lymphokines, or modified lymphokines, where appropriate by chromatography on a non-polar stationary phase.

2. The process as claimed in claim 1, wherein lymphokines or modified lymphokines from E. coli are isolated and purified.

3. The process as claimed in claim 1 or 2, wherein acetic acid or a mixture containing acetic acid is used.

4. The process as claimed in one of claims 1 to 3, wherein the extracted lymphokines or modified lymphokines are purified by HPLC on a derivatized silica gel for reverse-phase chromatography.

5. The process as claimed in claim 4, wherein the stationary phase used is a highly porous, alkylsilylated, aralkylsilylated or arylsilylated silica gel.

6. The process as claimed in claim 4 or 5, wherein the eluting agent used is an aqueous system with a gradient of a lower alkyl cyanide, preferably acetonitrile, or of a lower alcohol, preferably n-propanol, or of the mixture thereof, in a pH range from 1 to 7.

7. The process as claimed in one of claims 1 to 6, wherein interleukin-2 or modified interleukin-2 is isolated and purified.

8. The process as claimed in one of claims 1 to 7, wherein a peptide which has the amino acid sequence of human interleukin-2 is isolated and purified.

9. The process as claimed in one of claims 1 to 7, wherein a biologically active, modified lymphokine, preferably a biologically active modified interleukin-2, is isolated and purified, its amino acid sequence being modified by
   a) one or more amino acids having been replaced by other genetically codable amino acids and/or
   b) one or more amino acids being missing at the N-terminal end or
   c) one or more genetically codable amino acids being additionally present at the N-terminal end and/or
   d) one or more amino acids being missing at the C-terminal end or
   e) one or more genetically codable amino acids being additionally present at the C-terminal end.

10. The process as claimed in claim 8 or 9, wherein one or more amino acids in the sequence have been modified and/or substituted with physiologically tolerated groups.


**Revendications**

1. Procédé de séparation et de purification de lymphokines ou de lymphokines modifiées à partir de produits de désintégration des cellules provenant de cultures de bactéries modifiées par genié génétique, caractérisé en ce qu'on les extrait du dépôt de la désintégration cellulaire à l'aide d'un acide organique aliphatique inférieur ou un mélange composé de cette acide, d'eau, d'acétonitrile et (ou) d'un alcool aliphatique inférieur, et que les lymphokines ou lymphokines modifiées sont alors purifiées par chromatographie sur une phase stationnaire non polaire.

2. Procédé selon la revendication 1, caractérisé en ce que les lymphokines ou lymphokines modifiées sont séparées et purifiées à partir d'E.Coli.

3. Procédé selon les revendications 1 ou 2, caractérisé en ce qu'on utilise l'acide acétique ou un mélange contenant de l'acide acétique.

4. Procédé selon les revendications 1 à 3, caractérisé en ce que les lymphokines ou lymphokines modifiées sont purifiées par HPLC en chromatographie en phase inverse sur gel de silice greffé.

5. Procédé selon la revendication 4, caractérisé en ce qu'on utilise comme phase stationnaire un gel de silice de forte porosite, alkylsilylé, arylalkylsilylé ou arylsilylé.

6. Procédé selon les revendications 4 ou 5, caractérisé en ce qu'on utilise comme éluant, un système aqueux avec un gradiant d'un alcanenitrile inférieur, de préférence l'acétonitrile, ou d'un alcool inférieur, de préférence le n-propanol, ou d'un mélange des 2 dans un domaine de pH de 1 à 7.

7. Procédé selon les revendications 1 à 6, caractérisé en ce qu'on sépare et qu'on purifie l'interleukine-2 ou l'interleukine-2 modifiée.

8. Procédé selon les revendications 1 à 7, caractérisé en ce qu'on sépare et purifie un peptide qui présente la séquence des acides aminés de l'interleukine-2 humaine.

9. Procédé selon les revendications 1 à 7, caractérisé en ce qu'on sépare et purifie une lymphokine modifiée biologiquement active, de préférence une interleukine-2 modifiée biologiquement active, dont la séquence des acides aminés a été modifiée de la façon suivante :
   a) un ou plusieurs acides aminés ont été remplacés par d'autres acides aminés génétiquement codifiables et (ou)

b) un ou plusieurs acides aminés manquent à l'extrémité N-terminale ou

c) un ou plusieurs acides aminés codifiables génétiquement ont été ajoutés à l'extrémité N-terminale et (ou)

d) un ou plusieurs acides aminés manquent à l'extrémité C-terminale ou

e) un ou plusieurs acides aminés codifiables génétiquement ont été ajoutés à l'extrémité C-terminale.

10. Procédé selon les revendications 8 ou 9, caractérisé en ce que un ou plusieurs des acides aminés de la séquence sont transformés et/ou sont substitués par des groupes physiologiquement acceptables.